(19) Europäisches Patentamt
European Patent Office
Office européen des brevets

(11) **EP 2 170 154 B1**

(12) **EUROPÄISCHE PATENTSCHRIFT**

(45) Veröffentlichungstag und Bekanntmachung des Hinweises auf die Patenterteilung:
**27.02.2019 Patentblatt 2019/09**

(21) Anmeldenummer: **08784839.6**

(22) Anmeldetag: **17.07.2008**

(51) Int Cl.:
*A61B 3/00* *(2006.01)*     *A61B 3/113* *(2006.01)*
*A61B 5/16* *(2006.01)*     *A61B 3/024* *(2006.01)*

(86) Internationale Anmeldenummer:
**PCT/EP2008/005858**

(87) Internationale Veröffentlichungsnummer:
**WO 2009/010291 (22.01.2009 Gazette 2009/04)**

(54) **VERFHAREN UND VORRICHTUNG ZUM BEWERTEN DES GESICHTSFELDS**

METHOD AND DEVICE FOR ASSESSING THE FIELD OF VISION

PROCÉDÉ ET DISPOSITIF D'ÉVALUATION DU CHAMP VISUEL

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **17.07.2007 DE 102007033614**

(43) Veröffentlichungstag der Anmeldung:
**07.04.2010 Patentblatt 2010/14**

(73) Patentinhaber: **Heidelberg Engineering GmbH 69115 Heidelberg (DE)**

(72) Erfinder:
• **FLANAGAN, John, G.**
**Kitchener, Ontario N2M 2S9 (CA)**
• **CASSIDY, James**
**Waterloo, Ontario N2T 1V4 (CA)**

(74) Vertreter: **Wesch, Arno et al**
**Reble & Klose**
**Rechts- und Patentanwälte**
**Konrad-Zuse-Ring 32**
**68163 Mannheim (DE)**

(56) Entgegenhaltungen:
**EP-A- 0 962 188**     **EP-A- 1 397 991**
**EP-A- 1 442 695**     **WO-A-01/43637**
**DE-A1- 10 233 960**     **DE-A1- 19 649 858**
**US-A- 5 270 750**     **US-A1- 2002 047 987**

• **SUTTER ET AL: "The field topography of ERG components in man-I. The photopic luminance response" VISION RESEARCH, PERGAMON PRESS, OXFORD, GB, Bd. 32, Nr. 3, 1. März 1992 (1992-03-01), Seiten 433-446, XP022178094 ISSN: 0042-6989**

EP 2 170 154 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung bezieht sich auf ein Verfahren zur Bewertung des Gesichtsfelds gemäss den im Oberbegriff des Patentanspruchs 1 angegebenen Merkmalen. Ferner bezieht sich die Erfindung auf eine Vorrichtung zur Durchführung des Verfahrens.

Die US 2002/047987 A zeigt ein System zum Testen und Quantifizieren des Gesichtsfeldes gemäß dem Oberbegriff von Anspruch 1. Die EP 0 962 188 A1 offenbart ein Verfahren zum Messen der Sensitivitätsverteilung von Flimmern. Die US 5 270 750 A offenbart ein Verfahren zum Finden von blinden Flecken im Auge. Aus der EP 1 442 695 A2 ist bekannt, ein Gesichtsfeld dreidimensional abzubilden. Die DE 102 33 960 A1 zeigt ein Verfahren, bei dem Funktionsstörungen des Gehirns diagnostisch gesichert werden. Quaid und Flanagan untersuchen in Vision Research 45 (2005), S. 1075-1084, den Zusammenhang zwischen der Größe von Reizen (stimulus size) und der Zahl von zufälligen Punkten (random dots).

Aus der WO 01/60241 A1 sind beispielsweise ein Verfahren und eine Vorrichtung zur Untersuchung eines Auges bekannt, wobei eine als Kamera ausgebildete Bilderfassungseinheit und eine Beleuchtungseinheit in Verbindung mit einer Computer gestützten Bildauswertung zum Einsatz gelangt. Die Beleuchtungseinheit enthält eine Lichtquelle, wie beispielsweise einen Laser, sowie eine Ablenkungseinheit oder Scaneinheit, mittels welcher der Lichtstrahl der Lichtquelle in einer Ebene zweidimensional abgelenkt und zumindest Teilbereiche des Auges, beispielsweise die Oberfläche der Retina, abgetastet bzw. gescannt werden. Der reflektierte Lichtstrahl wird mittels der Bilderfassungseinheit erfasst und mittels des Computers erfolgt eine Auswertung der erfassten Daten, insbesondere durch die optische Kohärenz Tomographie (OCT-Technik), zur Darstellung von Flächenbildern mittels eines Bilderzeugungsgeräts. Die Vorrichtung bzw. das Ophthalmoskop enthalten geschlossene Regelkreise mit Motorsteuerung für ein Beleuchtungsoptik zwecks automatischer Fokuseinstellung für eine Abbildungsoptik. Des Weiteren ist die Helligkeit der Beleuchtungseinheit zwecks Erzielung eines guten Kontrastes und einer guten Ausleuchtung des abzubildenden Auges oder eines Bereiches desselben einstellbar.

Ferner ist ein Verfahren zur Gesichtsfelduntersuchung bekannt, welches auf dem Darstellen eines Stimulus bzw. Reizes basiert und in der Veröffentlichung "Ramachandran V. S., Rogers-Ramachandran D.: Phantom Contours: A New Class of Visual Patterns that Selectively Activates the Magnocellular Pathway in Man. Bulletin of the Psychonomic Society, 29, 391 (1991)" beschrieben ist. Das Verfahren bzw. die Darstellung des Reizes oder Stimulus ist bekannt als "flicker defined form" und wird nachfolgend als FDF bezeichnet,

Der Erfindung liegt die Aufgabe zugrunde, ein neues Verfahren und eine Vorrichtung vorzuschlagen, um effizient das Gesichtsfeld einer Person zu bewerten und/oder um insbesondere frühe Anzeichen von Erkrankungsprozessen zu erkennen, welche zu Einschränkungen des Gesichtsfeldes führen können. Ferner sollen das Verfahren sowie die Vorrichtung dahingehend ausgebildet und/oder optimiert werden, dass Ergebnisse der Gesichtsfelduntersuchung mit Ergebnissen einer strukturellen Bewertung des Sehnervkopfes eines Auges kombiniert werden, um die diagnostische Leistungsfähigkeit zu erhöhen, insbesondere für Erkrankungen, wie Glaukom, welche sowohl zu funktionalen Änderungen des Gesichtsfeldes als auch zu strukturellen Änderungen des Sehnervkopfes führen. Ferner liegt der Erfindung die Aufgabe zugrunde, das Verfahren und die Vorrichtung dahingehend weiterzubilden, dass in einfacher Weise die Untersuchung und/oder die Bewertung des Gesichtsfeldes durchgeführt werden kann und/oder dass die Entscheidungssicherheit optimiert wird. Weiterhin soll eine schnelle und zuverlässige Überprüfung und/oder Bewertung ermöglicht werden, wobei subjektive Bewertungen vermieden werden sollen.

Die Lösung dieser Aufgabe erfolgt hinsichtlich des Verfahrens gemäß den im Patentanspruch 1 angegebenen Merkmalen und ferner hinsichtlich der Vorrichtung gemäß den im Vorrichtungsanspruch angegebenen Merkmalen.

Das erfindungsgemäße Verfahren sowie die Vorrichtung ermöglichen eine vollständige und insbesondere automatische Bewertung unter Benutzung eines computergesteuerten Systems zum Darstellen von FDF-Reizen zur effizienten Bestimmung der Sehfunktion. Die Erfindung offenbart ein neues Verfahren und eine neue Vorrichtung und ermöglicht eine effiziente Bewertung insbesondere des Gesichtsfelds einer Person, um insbesondere frühe Anzeichen von Erkrankungsprozessen zu erkennen, welche zu Einschränkungen des Gesichtsfelds führen können. Erfindungsgemäß werden Ergebnisse der Gesichtsfelduntersuchung mit Ergebnissen der strukturellen Bewertung insbesondere des Sehnervkopfes kombiniert, wodurch die diagnostische Leistungsfähigkeit für Erkrankungen, wie beispielsweise Glaukom, zu erhöhen, welche sowohl zu funktionalen Änderungen des Gesichtsfelds als auch zu strukturellen Änderungen des Sehnervkopfs führen.

Erfindungsgemäß werden ein Verfahren zur Augenuntersuchung oder ein hierfür ausgebildetes Gerät oder System, wie Perimeter, konfokales Laser-Scanning-System, OCT-Interferometer oder Laserophthalmoskop (SLO), kombiniert mit dem FDF-Verfahren bzw. einer FDF-Einrichtung. Durch die erfindungsgemäße Kombination und/oder Integration zu einem einheitlichen Verfahren und/oder einer einheitlichen Vorrichtung werden in überraschend einfacher Weise aufgrund gleichzeitiger bzw. simultaner Erfassung ebenso wie Darstellung und/oder Untersuchungen und/oder Bewertungen von funktionalen und/oder strukturellen Änderungen des Auges ermöglicht. Der FDF-Reiz oder FDF-Stimulus besteht und/oder nutzt und/oder erfolgt mit einer Bilderzeugungseinheit, wie Monitor bzw. einer Kathodenstrahlröhre (CRT), Flachbildschirm, Projektionsgerät oder dergleichen und zwar insbesondere mit der gleichen gemeinsamen Bilderzeugungseinheit. Die Anwendung der FDF-Untersuchung durch das erfindungsgemäße Verfahren und/oder mittels der erfindungsgemäßen Vorrichtung ermöglichen in zuverlässiger Weise die Nutzung der FDF-Perimetrie, um Untersuchungen des Auges, insbesondere auf Glaukom, Sehnerv- und/oder Netzhauterkrankung und Abnormität sowie diabetische Augenerkrankung, neurologische Erkrankung und Abnormität, durchzuführen. Ferner können erfindungsgemäß Patienten mit geringer Sehkraft, Patienten mit speziellen Bedürfnissen und möglicherweise Patienten mit Lernbehinderung gemäß dem erfindungsgemäßen Verfahren und/oder mit der erfindungsgemäßen Vorrichtung untersucht werden.

In einer besonderen Ausgestaltung der Erfindung gelangen ein Verfahren und/oder eine Vorrichtung zur Bestimmung eines Artefakts und/oder des Beginns eines Reizes zur Korrektur oder Erfassung eines Artefakts und/oder zur Reizbeginn-Artefakt-Korrektur zum Einsatz und/oder zur Verwendung. Ein FDF-Reiz besteht aus und/oder nutzt eine Bilderzeugungseinheit (CRT-Anzeige, LCD-Anzeige, Projektionsgerät oder dergleichen), auf welcher viele kreisförmige Objekte oder Punkte auf einem festen Hintergrund angezeigt werden. Die aufzeichenbare Fläche der Bilderzeugungseinheit wird in bevorzugter Weise und/oder zumindest näherungsweise weitgehend und/oder vollständig mit diesen Punkten gefüllt. Die genannten Punkte sind in zwei Kategorien eingeordnet, nämlich Hintergrund und Ziel. Die Hintergrundpunkte decken bevorzugt den größten Teil der sichtbaren Anzeige ab. Ein kreisförmiger Bereich der Anzeigeeinheit und/oder der Bilderzeugungseinheit ist zur Darstellung des Reizes bestimmt und die Punkte, welche sich innerhalb des Reizbereiches befinden und/oder aufhalten, werden als Zielpunkte klassifiziert und/oder eingeordnet.

Während der Darstellung des FDF-Stimulus, welcher nachfolgend nur noch als FDF-Reiz bezeichnet wird, bewegen sich die Punkte zwischen zwei Helligkeiten bzw. Intensitätswerten bei einer festen Frequenz. Die erste Stufe ist ein fest gelegter Leuchtkraftwert oberhalb der Hintergrundleuchtkraft. Und die zweite Helligkeit ist derselbe Leuchtkraftwert unterhalb der Hintergrundleuchtkraft. Es ist allerdings wichtig anzumerken, dass sich die Hintergrundpunkte und Zielpunkte während der Reizdarstellung in Gegenphase befinden. Zum Beispiel, wenn die Zielpunkte heller sind als die Hintergrundleuchtkraft, sind die Hintergrundpunkte dunkler als die Hintergrundleuchtkraft und umgekehrt.

Eine andere besondere Ausgestaltung des erfindungsgemäßen Verfahrens und/oder der Vorrichtung ermöglicht die Bewertung einer Ermüdung der untersuchten Person durch Benutzung einer Echtzeit-Fixierungsüberwachung. Während der Ausführung der FDF-Untersuchung werden Ziele an verschiedenen Stellen relativ zu der optischen Achse des Instruments dargestellt. Um den starren Blick bzw. die Fixierung auf diesen Punkt zu unterstützen, wird ein kleiner schwarzer Punkt bereit gestellt (Fixierungsziel). Damit die Koordinaten der verschiedenen Zielexzentrizitäten korrekt sind, wird zudem ein Mittel bereit gestellt, um die Korrektheit der Fixierung während des Zeitraums, in dem das Ziel dargestellt wird, zu bestätigen.

Diese Bestätigung wird durch eine Bilderfassungseinheit, wie eine kleine CCD-Kamera, bereit gestellt, die das untersuchte Auge der Person entweder direkt oder über einen Strahlteiler, der in der optischen Achse zwischen der Person und der Anzeige angeordnet ist, betrachtet. Die Bilder werden durch den Computer verarbeitet, der Algorithmen anwendet, um den Punkt des starren Blicks der Person zu bestimmen und/oder die korrekte Fixierung zu erfassen. Dies wird erreicht durch das Identifizieren von charakteristischen Strukturen des Auges, zum Beispiel der Pupille, und durch ständiges Verfolgen der Position dieser Struktur während der FDF-Untersuchung.

Das Verfolgen wird durch das Analysieren des Bildes der Bilderzeugungseinheit bzw. der CCD-Kamera mit dem Computer ausgeführt. Wenn der Computer bestimmt und/oder mittels diesen festgestellt wird, dass die Person während der Zieldarstellung nicht fixiert war, werden die Ergebnisse einer solchen Erfassung und/oder Darstellung verworfen.

Außerdem können Informationen bezüglich des Ermüdungsgrades der Person von der Verlustrate der Fixierung abgeleitet werden. Diese Informationen können benutzt werden, um die Qualität der Untersuchungsergebnisse zu bewerten, oder um anzuzeigen, dass die Person eine Pause von der Untersuchung benötigt.

In einer weiteren besonderen Ausgestaltung des Verfahrens und/oder der Vorrichtung wird die Bewertung einer Patientenzuverlässigkeit durch Benutzung einer ständigen Reaktionszeitüberwachung ermöglicht. Das erfindungsgemäß entwickelte und/oder vorgeschlagene Verfahren und/oder die zur Durchführung desselben zum Einsatz gelangende Vorrichtung ermöglicht die ständige Überwachung der Reaktionszeit eines Patienten auf die Darstellung des FDF-Reizes.

Die Reaktionszeit wird gemessen als die Zeit, insbesondere in Millisekunden, zwischen der Auslösung einer Zieldarstellung und dem Drücken der Ansprechtaste durch den Patienten. Dies wird für jede Zieldarstellung während einer Untersuchung überwacht, und ein mitlaufender Durchschnitt wird berechnet, zusammen mit Vertrauensgrenzen. Jedes Ansprechen, das außerhalb des 95-zigsten Perzentils fällt, wird entweder als ein falsches positives (<) oder ein unzuverlässiges Ansprechen (>) gewertet.

Eine besondere Anwendung dieser Daten besteht darin, einzelne Reaktionszeitcharakteristiken und patientenbezogene Vertrauensgrenzen für Reaktionszeitverhalten festzulegen, um die Wahrscheinlichkeit zu bestimmen, ob ein Ansprechen ein falsches positives Ansprechen ist. Diese Information kann wiederum benutzt werden, um Zuverlässigkeitsparameter festzulegen.

In einer weiteren Ausgestaltung erfolgt gemäß dem Verfahren und/oder mit der Vorrichtung eine Randfelduntersuchung. Durch Zufügen von Lichtquellen, insbesondere von LEDs, welche körperlich und/oder optisch insbesondere im horizontalen Randfeld angeordnet sind. ergibt sich die einzigartige und/oder besondere Fähigkeit, um insbesondere für Fahrtauglichkertsprüfung oder für möglicherweise besondere neurologische Gebiete zu messen und/oder zu bewerten.

In einer besonderen Weiterbildung und/oder Ausgestaltung des Verfahrens und/oder der Vorrichtung sind diese zur Steuerung einer erhöhten Helligkeitsauflösung ausgebildet. StandardGrafikkartentechnologie stellt $2^8$, oder 256 Stufen an. Helligkeit für jeden der roten, grünen und blauen Farbkanäle bereit. Es wurde eine Technik entwickelt, bei der ein Schwarzweißbildschirm (CRT, LCD oder ein Projektionsgerät) zwei der Farbkanäle benutzt, um Helligkeitsstufen mit bis zu $2^{16}$ oder 65536 Helligkeitsstufen bereit zu stellen.

Das Verfahren und/oder die Vorrichtung benutzen insbesondere die grünen und roten Kanäle eines RGB-Signals mit 8 Bits Helligkeitssteuerung pro Kanal bevorzugt derart, dass die daraus folgende Steuerung ein Schwarzweißkanal mit 16 Bits Intensitäts- oder Heilligkeitsregulierung ist. Der grüne Kanal wird als die 8 Bits mit dem höchsten Stellenwert benutzt. Daher sorgt eine Stufe des grünen Kanals für ein Helligkeitsinkrement von 1/256 des Maximums. Der rote Kanal wird als die 8 Bits mit dem niedrigsten Stellenwert benutzt. Daher sorgt eine Stufe des roten Kanals für ein Heilligkeitsinkrement von 1/65536 des Maximums. Die resultierenden zwei Signale werden addiert mit der resultierenden Intensitäts- oder Helligkeitssteuerungsfunktion:

$$\text{Helligkeit} = \text{maximale Helligkeit} \cdot \left[ \frac{G}{256} + \frac{R}{65536} \right]$$

wobei G die grüne Signalstufe ist, deren Werte von 0 bis 255 reichen, und R die rote Signalstufe Ist, deren Werte von 0 bis 255 reichen

Obwohl diese Technik für ein theoretisches Maximum von 65536 Helligkeitsstufen sorgt, ist in der Praxis die Anzahl der Steuerungsstufen, die erreicht werden können, wesentlich geringer, irgendwann wird die Änderung in der Signalstufe des kombinierten Helligkeitssteuerungsslgnals geringer sein als das Rauschen, das immer im System vorhanden ist.

Der Vorteil dieser Technik ist, dass wenn eingeführt, die steuernde Software gestaltet werden kann, um jede Anzahl von Helligkeitssteuerungsstufen, von $2^8$ bis $2^{16}$ reichend, bereitzustellen, Dies wird erreicht, indem die 8 Bit mit dem höchsten Stellenwert des erforderlichen Helligkeitswerts in dem grünen Farbkanal angeordnet werden. Die übrigen Bits des erforderlichen Helligkeitswerts werden in den Bits mit dem höchsten Stellenwert des roten Kanals angeordnet.

$$\text{Gruen} = \frac{\left( \text{Helligkeit}_{16} \wedge 1111111100\,000000\,B \right)}{11111111\,B}$$

$$\text{Rot} = \left( \text{Helligkeit}_{16} \wedge 0000000011111111\,B \right) x 11111111\,B$$

In einer weiteren Ausgestaltung gelangt ein adaptiver stufenförmiger Schwellenwertalgorithmus (ASTA) zum Einsatz, welcher ein Schwellenwert-Schätzalgorithmus ist, der einige besondere Techniken verwendet.

- Jede Zielposition kann unterschiedlichen Strategien von Stufenformen zugewiesen werden.
- Ein schneller Ablauf wird jeder Position zugewiesen, die eine einzelne Schwellenwertüberschreitung innerhalb des 95-ten Perzentils der altersbedingten normalen Werte der Reizposition durchläuft. Der Startwert für jeden psychophysikalischen stufenförmigen Schwellenwert-Schätzalgorithmus wird von dessen Nachbarn gesetzt und entwickelt sich aus vier anfänglichen Setzpunkten, die alle einen vollständigen Stufenform (4-2-2) -Ablauf durchlaufen. Der Startwert für alle diese zweitrangigen Punkte ist eine vorbestimmte Anzahl von Dezibeln unterhalb des benachbarten Schwellenwerts. Eine 2-2-Stufenform wird benutzt, aber wenn die erste Schwellenwertüberschreitung in der aufsteigenden Richtung und innerhalb des 95-ten Vertrauensintervalls gesehen wird, dann ist die Schwellenwertschätzung abgelaufen.
- Altersbedingte normale Daten werden gebildet, indem nichtlineare Verfahren für jeden Reizort benutzt werden. Es werden ebenfalls nichtlineare Vertrauensgrenzen festgelegt. Dies stellt eine erhöhte Genauigkeit für die Schwellenwert-Schätzmuster und die Datenanalyse bereit.
- Die Geschwindigkeit und Genauigkeit des Algorithmusses können durch Anpassen des für einen schnellen Ablauf erlaubten Vertrauensintervalls verändert werden. Zum Beispiel wird sich die Geschwindigkeit erhöhen und die Genauigkeit abnehmen, wenn größere Vertrauensgrenzen für das Kriterium des schnellen Ablaufs benutzt werden.

- Es gibt zwei Arten der Untersuchung: i. Erster ASTA (iASTA), der benutzt wird, um Ausgangsdaten festzulegen. ii. ASTA, der Informationen von vorangehenden Feldern (iASTA oder ASTA, ein Einzelnes oder der Durchschnitt von mehreren Ausgangsfeldern) benutzt, um effizient den Stufenform-Algorithmus voranzutreiben. iASTA benutzt eine vollständige Schwellenwert-Bestimmungsstrategie. ASTA benutzt dann die vorbestimmten Ausgangsdaten, um die Startwerte für jede Reizposition zu setzen und reduziert dabei die Untersuchungszeit.

Des Weiteren gelangt bevorzugt eine Sprunggrenzenstrategie zum Einsatz und/oder zur Verwendung. Angesichts der Art des zufälligen Punkthintergrunds, wenn der FDF-Reiz erzeugt wird, ist es wichtig, die Kontraständerung zwischen aufeinanderfolgenden Darstellungen zu begrenzen. Insbesondere auf Erfahrungswerten basierende Grenzen werden für jede Kontraststufe vorgegeben und ein Verfahren zum Aufnehmen dieser maximal zulässigen Stufen in den Schwellenwert-Schätzalgorithmus (bevorzugt ASTA) gelangt zum Einsatz. Die Grenzen sind empirisch bestimmt und variieren hinsichtlich und/oder abhängig der Exzentrizität und der Defekttiefe. Zum Beispiel ist oder wird die Grenze für einen Reizkontrast von 24dB, der bei einer Exzentrizität von 3 Grad dargestellt wird, mit 4dB vorgegeben.

In einer anderen besonderen Ausgestaltung erfolgt ein Alterungsabgleich, insbesondere des CRT Phosphor, durch automatische Steuerungskorrektur. Das FDF-Instrument bzw. die FDF-Einheit erfordert, dass die Helligkeit des dargestellten Reizes richtig abgestimmt bleibt. Wird allerdings eine CRT benutzt, nimmt die Leuchtkraft des Phosphors für eine bestimmte Steuerungseinstellung mit der Zeit ab. Auf ähnliche Weise kann sich die Leuchtkraft einer LCD-Einheit oder die Helligkeit eines Projektionsgeräts oder allgemein der Bilderzeugungseinheit mit der Zeit ändern.

Die FDF-Einheit enthält eine Schaltungstechnik, die ein Mittel zum Messen der Helligkeit des durch die Bilderzeugungseinheit, wie CRT oder LCD-Einheit oder Projektionsgerät, erzeugten Lichts bereitstellt. Die FDF-Einheit misst periodisch die Helligkeit des durch die Bilderzeugungseinheit erzeugten Lichts über ihren gesamten Steuerungsbereich. Durch Überwachen der Leuchtkraft der Bilderzeugungseinheit und Steuern des Steuerungswerts durch einen Mikroprozessor, wird dieses Verhalten automatisch korrigiert.

Die Schaltungstechnik enthält ein Paar von Photodioden, welches das Licht von der Bilderzeugungseinheit, wie der CRT oder der LCD oder dem Projektionsgerät, abtastet. Das Signal von jeder Photodiode steuert einen Strom zum Spannungswandler, dann einen Verstärker mit programmierbarer Verstärkung. Die programmierbare Verstärkung wird benutzt, um einzelne Empfindlichkeiten der Photodioden abzugleichen. Das aufbereitete Signal wird dann mit einem ADC abtastet. Zum Zeitpunkt der Herstellung wird eine Tabelle erzeugt, um einen Zusammenhang zwischen dem ADC-Zählwert und cd/m$^2$ bereitzustellen. Im Wesentlichen stellt die Schaltungstechnik ein Instrument mit eingebautem Belichtungsmesser bereit.

Ganz offensichtlich kann das Altern der Photodiode selbst mit der Zeit Änderungen der Messanzeigen der Photodiode verursachen. Um dies auszuschließen, wird ein Paar von Photodioden benutzt, wie oben beschrieben. Nur wenn beide Photodioden ähnliche Messanzeigen erzeugen, wird die Messung der Leuchtkraft angenommen. Andernfalls wird der Bediener informiert, dass die Vorrichtung eine Überprüfung oder Instandsetzung benötigt.

In einer besonderen Ausgestaltung der Erfindung ist ein Verfahren zum Bestimmen der korrekten Position des Sehnervkopfes in dem Gesichtsfeld und dessen Benutzung vorgesehen. Um den Bereich des Gesichtsfelds genau bestimmen zu können, der einem bestimmten Abschnitt des Sehnervkopfes entspricht und umgekehrt, ist es wichtig, die relative Position des Sehnervkopfes zu der Fovea genau zu kennen. In einer Überprüfung, beispielsweise mit einem Retina Tomograph fixiert der Patient ein Fixierungsziel, das eine feste und bekannte Position relativ zu der optischen Achse des Instruments aufweist. Alle erhaltenen Bilder sind mittig um die optische Achse des Instruments angeordnet. Dadurch lässt die Mittenposition des Sehnervkopfes innerhalb eines erhaltenen Bildes die genaue Bestimmung der relativen Position des Sehnervkopfes zu der Fovea zu.

Mit dieser Kenntnis ist es möglich, um

- entweder während der Gesichtsfelduntersuchung den Reiz an den korrekten Positionen derart darzustellen, dass ein bestimmter Bereich des Gesichtsfelds genau einem bestimmten Abschnitt des Sehnervkopfes entspricht,
- oder die Gesichtsfelduntersuchungsergebnisse nach der Gesichtsfelduntersuchung derart nachzuzeichnen, dass eine korrekte Zuordnung von bestimmten Bereichen des Gesichtsfelds zu bestimmten Abschnitten des Sehnervkopfes aufrechterhalten wird.

In der erfindungsgemäßen Vorrichtung sind zusätzlich zur Kombination und/oder Integration der FDF-Einheit in die Vorrichtung bzw. das Gerät, mit welchem Strukturen des Auges untersucht und/oder erfasst und/oder bewertet werden, die vorstehend erläuterten Verfahrensmaßnahmen oder Funktionsweisen einzel oder einige oder auch sämtliche kombiniert und mittels des Computers, sei es als Hardware oder als Software realisiert. Weiterhin umfasst die Erfindung die Verwendung der erfindungsgemäß vorgeschlagenen und/oder ausgebildeten Vorrichtung.

Besondere Ausgestaltungen und Weiterbildungen der Erfindung, und zwar sowohl des Verfahrens als auch der Vorrichtung, sind in der Zeichnung dargestellt und nachfolgend beschrieben, wobei insoweit keine Beschränkung erfolgt. Es zeigen:

Fig. 1    Diagramme der Hintergrundpunkte und der Zielpunkte der Bilderzeugungseinheit zur Bestimmung eines Artefakts,

Fig. 2    eine Darstellung des Sehnervkopfs und des Gesichtsfelds,

Fig. 3    ein Bild des Sehnervkopfs entsprechend der strukturellen Klassifizierung,

Fig. 4    Darstellungen der Gesichtsfeldergebnisse,

Fig. 5    eine weitere Darstellung der Gesichtsfeldergebnisse.

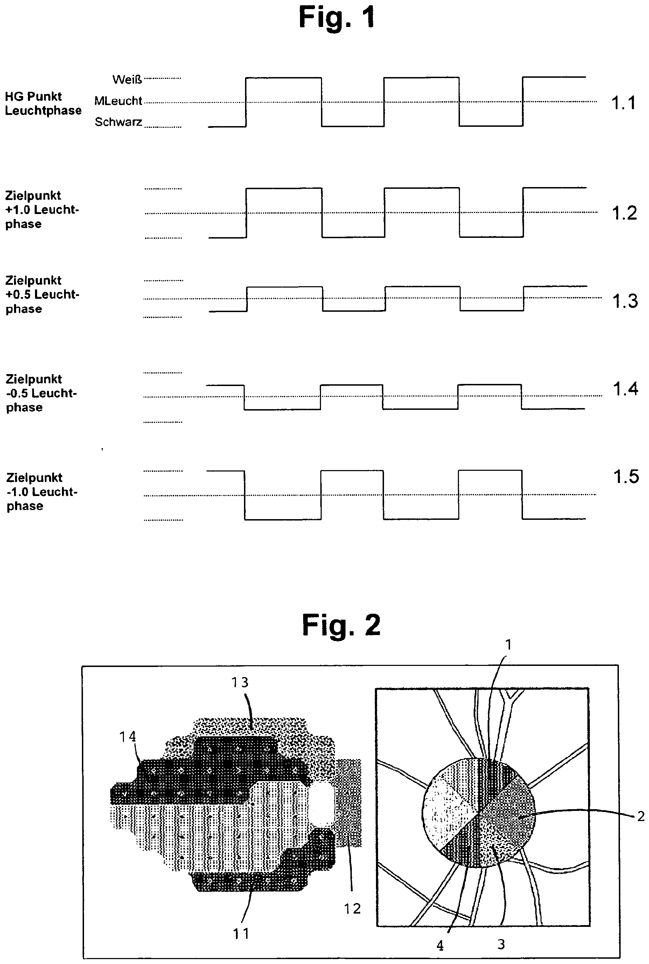

Anhand von Fig. 1 wird die Bestimmung eines Artefakts oder der Reizbeginn zur Korrektur oder Erfassung eines Artefakts erläutert. Ein FDF-Reiz nutzt die Bilderzeugungseinheit, auf welcher eine Vielzahl kleiner kreisförmiger Objekte oder Punkte auf einem soliden Hintergrund angezeigt werden. Die aufzeichenbare und/oder nutzbare Fläche der Bilderzeugungseinheit wird bevorzugt zumindest näherungsweise vollständig mit derartigen Punkten gefüllt. Diese Punkte sind in zwei Kategorien klassifiziert, nämlich Hintergrundpunkte und Zielpunkte. Die Hintergrundpunkte bedecken bevorzugt den größten Teil der sichtbaren Anzeigeeinheit. Ein kreisförmiger Bereich der Anzeigeeinheit ist zur Abbildung des Reizes oder Stimulus vorgesehen und diejenigen Punkte, welche sich innerhalb des Bereichs des Stimulus oder Reizes befinden, sind als Zielpunkte klassifiziert. Während der Darstellung des FDF-Stimulus bzw. des FDF-Reizes bewegen sich die Punkte zwischen zwei Helligkeits- bzw. Intensitätswerten mit vorgegebener fester Frequenz hin und her. Hierbei ist die erste Stufe bzw. ein erster Level ein festgelegter Helligkeits- oder Leuchtkraftwert oberhalb der Hintergrundhelligkeit oder Hintergrundleuchtkraft. Die zweite Helligkeit oder Intensität weist denselben Wert der Leuchtkraft oder Helligkeit unterhalb der Hintergrundleuchtkraft auf. Insoweit sei angemerkt, dass die Hintergrundpunkte und die Zielpunkte während der Darstellung des Reizes oder Stimulus sich in Gegenphase befinden. Wenn z.B. die Zielpunkte heller sind als die Hintergrundleuchtkraft, so sind die Hintergrundpunkte dunkler als die Hintergrundleichtkraft und umgekehrt. Wenn nun die Hintergrundpunkte und die Zielpunkte in Phase sind, scheint die Anzeigeeinheit oder Bilderzeugungseinheit homogen zuflimmern. Wenn hingegen die Hintergrundpunkte und die Zielpunkte in Gegenphase sind, erscheint ein Schatten ähnlicher Ring um das Perimeter und/oder den Außenbereich des Zielpunktbereichs.

Wenn ein FDF-Reiz oder Stimulus dargestellt wird, resultiert der plötzliche Wechsel der Reizpunkte vom Zustand in Phase mit den Hintergrundpunkten zum Zustand 180° Phase verschoben in einem Artefakt, wobei der Reiz vorübergehend heller erscheint als das umgebende Punktefeld. Die Grundlage des Artefakts ist physiophysischer Natur und beruht nicht auf abnormen oder anormalen Helligkeitsstufen. Dasselbe Artefakt tritt ebenfalls bei Reizverschiebung auf.

Das Verfahren bzw. die Technik, welche benutzt wird, um dieses Artefakt zu korrigieren, umfasst den allmählichen oder graduellen Übergang der Zielpunktumkehrung vom Zustand in Phase zu den Hintergrundpunkten in phasenverhobenen Zustand. Beginnend mit der Umkehrung des phasengleichen Zustands der Zielpunkte mit den Hintergrundpunkten wird die Amplitude der Zielpunktumkehrung in mehreren Schritten verringert, bis die Amplitude den Wert Null erreicht. Die Amplitude der Zielpunkte steigt dann an, wenn sich die Spitzenstärke in Gegenphase mit den Hintergrundpunkten befindet.

Dieser Verlauf ist im Diagramm der Fig. 1 dargestellt. Die erste Wellenform 1.1 stellt den Zyklus der Hintergrundpunkte zwischen Werten der weißen und schwarzen Leuchtkraft bzw. zwischen weißen und schwarzen Helligkeitswerten in einer symmetrischen Art und Weise um die mittlere Leuchtkraft bzw. mittlere Helligkeit dar. Die nächsten vier Wellenformen 1.2 bis 1.4 zeigen den Verlauf der Helligkeit der Zielpunkte bzw. der Zielpunktleuchtkraft von phasengleich bis 180° phasenverschoben relativ zur Leuchtkraft oder Helligkeit der Hintergrundpunkte.

Anhand von Fig. 2 wird eine besondere Ausgestaltung der Erfindung, nämlich eine Struktur-Funktionsanalyse, näher erläutert. Mehrere Erkrankungen des hinteren Augensegments, zum Beispiel Glaukom, führen sowohl zu Änderungen im Gesichtsfeld (funktionale Änderungen) als auch zu Änderungen in der Struktur des Sehnervkopfes (strukturelle Änderungen). Um solche Erkrankungen zu diagnostizieren oder den Fortschritt solcher Erkrankungen zu erkennen, ist es wichtig, die Funktion wie auch die Struktur zu bewerten.

Die erfindungsgemäße Kombination der FDF-Einheit, beispielsweise eines FDF-Perimeters, mit einem Gerät oder einer Vorrichtung, mit welcher Strukturen des Auges untersucht und/oder erfaßt und/oder bewertet werden, insbesondere die Sehnervkopfstruktur, zum Beispiel ein Retina Tomograph, ermöglicht eine einzigartige kombinierte Analyse von Struktur und Funktion. Dies basiert auf der bekannten Zuordnung von bestimmten Abschnitten, insbesondere des Sehnervkopfs, zu bestimmten Bereichen des Gesichtsfelds.

Fig. 2 zeigt rechts den Sehnervkopf unterteilt in sechs verschiedene Abschnitte 1 bis 6 und links das Gesichtsfeld unterteilt in sechs verschiedene Bereiche 11 bis 16. Der Abschnitt 1 entspricht dem Bereich 11, der Abschnitt 2 dem Bereich 12 usw. Die Sehnervkopfabschnitte und die Gesichtsfeldbereiche die einander entsprechen, weisen die gleiche insbesondere farbige Kodierung auf. So ist beispielsweise der Abschnitt 1 rot kodiert und ebenso der Bereich 11, ferner der Abschnitt 2 grün kodiert wie auch der Bereich 12 und weiterhin die anderen einander zugeordneten Abschnitte und Bereiche mit jeweils übereinstimmenden farbigen Kodierungen. Auch können, wie in der schwarz-weiß Darstellung der Fig. 2, die Abschnitte des Sehkopfnervs und die jeweils zugeordneten Bereiche der Gesichtsfelddarstellung mit übereinstimmenden graphischen Mustern kodiert sein. Ferner kann die Anzahl der Abschnitte und Bereiche nach den jeweiligen Anforderungen vorgegeben sein.

Jeder Abschnitt des Sehnervkopfs und jeder Bereich des Gesichtsfelds wird in eine von zwei oder mehreren Kategorien eingeteilt. Zum Beispiel kann ein Satz von drei Kategorien "innerhalb normaler Grenzen", "an der Grenze" und "außerhalb normaler Grenzen" sein. Um effektiv diese Resultate dem Untersuchenden, insbesondere dem Arzt, darzustellen, gelangen die nachfolgend erläuterten Verfahren zur Verwendung.

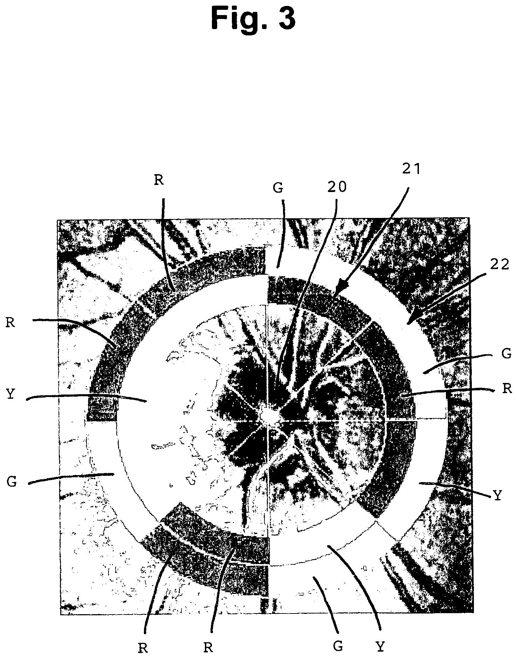

Ein erstes Verfahren wird an Hand von Fig. 3 erläutert, in welchem ein Bild des Sehnervkopfs 20 dargestellt und in zwei oder mehrere Abschnitte, hier acht Abschnitte, unterteilt ist. Zwei Ringe 21, 22 werden eingeblendet, die in dieselben Abschnitte unterteilt sind. Der innere Ring 21 zeigt das Ergebnis der strukturellen Klassifizierung für jeden Abschnitt des Sehnervkopfs durch einen Farbcode an, zum Beispiel Grün G für "innerhalb normaler Grenzen", Gelb Yfür "an der Grenze", Rot R für "außerhalb normaler Grenzen". Der äußere Ring zeigt im selben Farbcode das Klassifizierungsergebnis für den Bereich des Gesichtsfelds an, der einem Abschnitt des Sehnervkopfs entspricht.

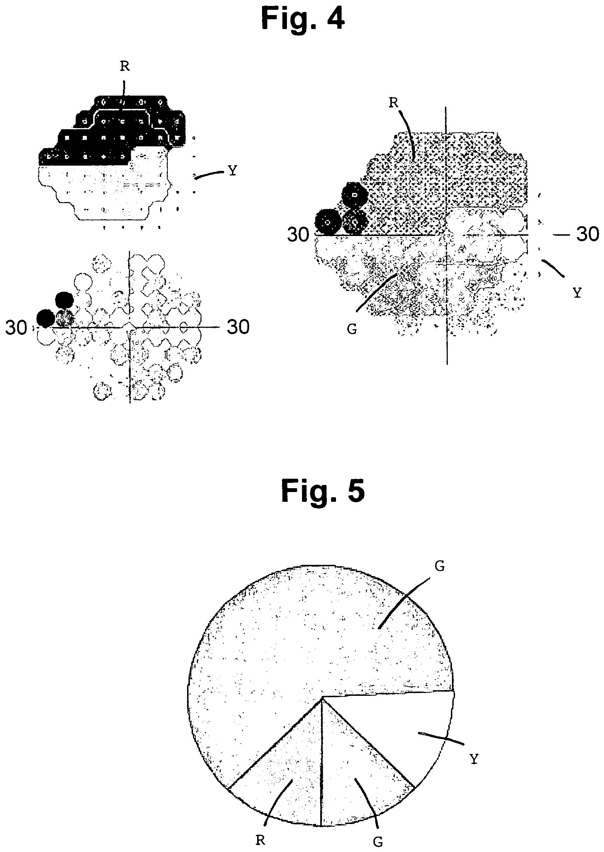

An Hand von Fig. 4 wird ein zweites Verfahren erläutert, wobei das Gesichtsfeldergebnis dargestellt wird durch Anzeige einer Abbildung mit dem Gesichtsfeldergebnis für jede Reizposition. Das gesamte Gesichtsfeld wird unterteilt in zwei oder mehr Bereiche, die bestimmten Abschnitten des Sehnervkopfes entsprechen. Der Hintergrund jedes Gesichtsfeldbereichs ist farbcodiert, wie vorstehend erläutert, entsprechend dem Klassifizierungsergebnis der Sehnervkopfstruktur, die diesem Bereich entspricht.

Außerdem können in einem dritten Verfahren die beiden durch die strukturelle Bewertung und die Gesichtsfeldbewertung bereitgestellten Messungen kombiniert werden bezüglich der Schätzungen von normaler, altersbedingter Ganglienzellendichte. Dies kann aufgezeichnet werden durch Uhrzeigerpositionen von Sehnervkopfabschnitten, in einem ähnlichen Stil wie die bekannte TSNIT-Aufzeichnung, mit der Ganglienzellendichte als die Ordinate, und beide Messungen von Struktur und Funktion aufgezeichnet pro Abschnitt auf der Abszisse.

Schließlich können in einem vierten Verfahren gemäß Fig. 5 die Gesichtsfeldergebnisse in ein Bild des Sehnervkopfes derart eingeblendet werden, dass jeder Sehnervkopfabschnitt gemäß dem Gesichtsfeldergebnis in seinem entsprechenden Bereich des Gesichtsfelds farbcodiert ist.

Die an Hand der Zeichnung erläuterten Besonderheiten und Weiterbildungen sind für den Bereich des Sehkopfnerves abgehandelt. Im Rahmen der Erfindung können diese Besonderheiten und Weiterbildungen ebenso wie die zuvor abgehandelten analog für andere Bereiche des Auges, wie der Makula, oder für andere Bereiche der Retina vorgesehen werden.

**Patentansprüche**

1. Verfahren zum Bewerten des Gesichtsfelds, wobei FDF-Reize dargestellt werden und von einer Person mit dem wenigstens einen zu untersuchenden Auge erfasst werden, wobei zur effizienten Bestimmung der Sehfunktion die Darstellung der FDF-Reize durch Benutzung eines Computer gesteuerten Systems durchgeführt wird, wobei der jeweilige FDF-Reiz durch Benutzung einer Bilderzeugungseinheit erzeugt wird, wobei die funktionalen Änderungen des Auges bewertet und/ oder erfasst werden, wobei eine Kombination mit einem Verfahren zur Erfassung von Strukturen und/ oder strukturellen Änderungen des Auges erfolgt, wobei eine Struktur-Funktionsanalyse durchgeführt wird und wobei zum einen die Struktur oder ein Teil der Struktur des Auges bewertet wird und zum anderen eine Zuordnung von vorgegebenen Abschnitten der Struktur zu bestimmten und/ oder vorgegebenen Bereichen des Gesichtsfelds erfolgt,

   **dadurch gekennzeichnet, dass**

   - jeder Abschnitt des Sehnervkopfs (20) oder einer anderen Struktur und jeder Bereich des Gesichtsfelds in eine von zwei oder mehreren Kategorien eingeteilt wird,
   - wobei ein Bild des Sehnervkopfs (20) oder der anderen Struktur dargestellt und in zwei oder mehrere Abschnitte unterteilt wird,
   - wobei zwei Ringe (21, 22) eingeblendet werden, die in dieselben Abschnitte unterteilt sind,
   - wobei ein innerer Ring (21) das Ergebnis der strukturellen Klassifizierung für jeden Abschnitt des Sehnervkopfs (20) oder der anderen Struktur durch einen Farbcode, graphische Muster oder Helligkeitsstufen anzeigt und
   - wobei ein äußerer Ring (22) im selben Farbcode, übereinstimmenden graphischen Muster oder Helligkeitsstufen das Klassifizierungsergebnis für den Bereich des Gesichtsfelds anzeigt, der einem Abschnitt des Sehnervkopfs (20) oder der anderen Struktur entspricht.

2. Verfahren nach Anspruch 1, **dadurch gekennzeichnet, dass** jeder Abschnitt des Sehnervkopfes (20) und jeder Bereich des Gesichtsfelds in eine der drei Kategorien "innerhalb normaler Grenzen", "an der Grenze" und "außerhalb normaler Grenzen" eingeteilt wird und/ oder dass der Farbcode G für "innerhalb normaler Grenzen", der Farbcode Y für "an der Grenze" und der Farbcode R für "außerhalb normaler Grenzen" steht.

3. Verfahren nach Anspruch 1 oder 2, **dadurch gekennzeichnet, dass** der dargestellte FDF-Reiz einer Artefaktkorrektur unterzogen wird, wobei ein allmählicher Übergang einer Zielpunktumkehrung vom Zustand in Phase zum zu den Hintergrundpunkten phasenverschobenen Zustand durchgeführt wird.

4. Verfahren nach einem der Ansprüche 1 bis 3, **dadurch gekennzeichnet, dass** eine Echtzeit-Fixierungsüberwachung, insbesondere zum Bewerten einer Ermüdung der Person, durchgeführt wird, wobei während der Ausführung der FDF-Untersuchung Ziele an verschiedene Stellen relativ zur optischen Achse des Instruments oder der Bilderzeugungseinheit zur Fixierung des Auges bereitgestellt werden, wobei die Korrektheit der Fixierung in einem vorgegebenen Zeitraum zu bestätigen ist.

5. Verfahren nach einem der Ansprüche 1 bis 4, **dadurch gekennzeichnet, dass** durch Benutzen einer ständigen Reaktionszeitüberwachung eine Bewertung der Zuverlässigkeit der Person bzw. des Patienten erfolgt, wobei insbesondere in einer vorgegebenen Zeit zwischen der Auslösung einer Zieldarstellung und der Betätigung einer Ansprechtaste als Reaktionszeit vorgegeben und/oder überwacht wird.

6. Verfahren nach einem der Ansprüche 1 bis 5, **dadurch gekennzeichnet, dass** eine Randfelduntersuchung vorgenommen wird, insbesondere mittels körperlich und/oder optisch in einem horizontalen Randfeld angeordneten Lichtquellen, und/oder dass ein Verfahren zur Steuerung einer erhöhten Helligkeitsauflösung zum Einsatz gelangt, insbesondere durch Verwendung einer Standardgrafikkartentechnologie, wobei für die Bilderzeugungseinheit, insbesondere ein Schwarz-Weiß-Bildschirm, zwei Farbkanäle zur Bereitstellung der vorgegebenen Anzahl von Helligkeitsstufen benutzt werden.

7. Verfahren nach einem der Ansprüche 1 bis 6, **dadurch gekennzeichnet, dass** ein adaptiver stufenförmiger Schwellenwert-Algorithmus (ASTA), insbesondere zur Zuweisung jeder Zielposition verwendet wird.

8. Verfahren nach einem der Ansprüche 1 bis 7, **dadurch gekennzeichnet, dass** eine Sprunggrenzenstrategie durchgeführt wird, wobei hinsichtlich des jeweiligen Punkthintergrunds eine Kontraständerung zwischen aufeinander folgende Darstellungen begrenzt wird.

9. Vorrichtung zur Durchführung des Verfahrens nach einem der Ansprüche 1 bis 8, **dadurch gekennzeichnet, dass** ein Computersystem und eine von diesem ansteuerbare Bilderzeugungseinheit zur Darstellung der FDF-Reize vorgesehen sind.

10. Vorrichtung zur Durchführung des Verfahrens nach Anspruch 1, **dadurch gekennzeichnet, dass** für die Erfassung der Strukturen und/oder strukturellen Änderungen eine Lichtquelle und eine Abtasteinrichtung vorgesehen sind und elektrisch mit dem genannten Computer sowie der genannten Bilderzeugungseinheit aus Anspr. 9 zur Datenübertragung in bekannter Weise verbunden sind.

**Claims**

1. A method for assessing the field of vision, wherein FDF stimuli are displayed and perceived by a person having the at least one eye to be examined; the display of the FDF stimuli is carried out using a computer-controlled system to determine visual function efficiently; each FDF stimulus is generated using an image generation unit; the functional changes in the eye are assessed and/or sensed; a combination with a method for sensing structures and/or structural changes in the eye is carried out; a structural-functional analysis is carried out, wherein firstly the structure or a part of the structure of the eye is assessed and secondly specified sections of the structure are assigned to defined and/or specified regions of the field of vision,
**characterised in that**

- each section of the optic nerve papilla (20) or of another structure and each region of the field of vision are allocated to one of two or more categories,
- wherein an image of the optic nerve papilla (20) or of the other structure is displayed and divided into two or more sections,

- wherein two rings (21, 22) are displayed and divided into the same sections,
- wherein an inner ring (21) indicates the result of the structural classification for each section of the optic nerve papilla (20) or of the other structure by means of a colour code, graphical patterns or brightness gradations, and
- wherein an outer ring (22) in the same colour code, corresponding graphical patterns or brightness gradations indicates the classification result for the region of the field of vision corresponding to a section of the optic nerve papilla (20) or of the other structure.

2. The method according to claim 1, **characterised in that** each section of the optic nerve papilla (20) and each region of the field of vision is allocated to one of the three categories "within normal limits", "at the limit" and "outside normal limits" and/or that the colour code G stands for "within normal limits", the colour code Y stands for "at the limit", and the colour code R stands for "outside normal limits".

3. The method according to claim 1 or 2, **characterised in that** the displayed FDF stimulus undergoes artefact correction, wherein a gradual transition of a target point reversal from the state in phase to the state phase-shifted relative to the background points is carried out.

4. The method according to any one of claims 1 to 3, **characterised in that** real-time fixation monitoring is carried out, in particular to assess fatigue of the person, wherein, while the FDF examination is being carried out, targets are provided at different locations relative to the optical axis of the instrument or of the image generation unit for fixation of the eye, wherein the correctness of the fixation must be confirmed within a specified time period.

5. The method according to any one of claims 1 to 4, **characterised in that** an assessment of the reliability of the person or patient is carried out using continuous reaction time monitoring, wherein in particular a specified time between the triggering of a target display and the operation of a response key is specified and/or monitored as the reaction time.

6. The method according to any one of claims 1 to 5, **characterised in that** a peripheral field examination is performed, in particular by means of light sources arranged physically and/or optically in a horizontal peripheral field, and/or that a method for controlling an increased brightness resolution is used, in particular using standard graphics card technology, wherein for the image generation unit, in particular a black and white screen, two colour channels are used to provide the specified number of brightness gradations.

7. The method according to any one of claims 1 to 6, **characterised in that** an adaptive step threshold algorithm (ASTA) is used, in particular for assigning each target position.

8. The method according to any one of claims 1 to 7, **characterised in that** a hop limit strategy is carried out, wherein, with regard to the point background in question, a change in contrast between successive displays is limited.

9. A device for carrying out the method according to one of claims 1 to 8, **characterised in that** a computer system and an image generation unit which can be controlled by said computer system for displaying the FDF stimuli are provided.

10. The device for carrying out the method according to claim 1, **characterised in that** to sense the structures and/or structural changes, a light source and a scanning device are provided and connected electrically to the said computer and to the said image generation unit from claim 9 for data transmission in a known manner.

**Revendications**

1. Procédé, destiné à évaluer le champ visuel, des stimuli FDF étant représentés et détectés par une personne à l'aide de l'au moins un oeil qui doit être examiné, pour une détermination plus efficace de la fonction visuelle, la représentation des stimuli étant réalisée par utilisation d'un système commandé par ordinateur, le stimulus FDF en question étant généré par utilisation d'une unité de génération d'images, les modifications fonctionnelles de l'oeil étant évaluées et/ou détectées, une association avec un procédé de détection de structures et/ou de modifications structurelles de l'oeil ayant lieu, une analyse fonctionnelle de structure étant réalisée et d'une part la structure ou une partie de la structure de l'oeil étant évaluée et d'autre part, une affectation de segments prédéfinis de la structure à des zones déterminées et/ou prédéfinies du champ visuel étant effectuée,
**caractérisé**

- **en ce qu'**on divise chaque segment de la tête du nerf optique (20) ou d'une autre structure et chaque zone du champ visuel en une de deux ou plusieurs catégories,
- une image de la tête du nerf optique (20) ou de l'autre structure étant représentée et divisée en deux ou en plusieurs segments,
- deux anneaux (21, 22) étant superposés qui sont divisés dans les mêmes segments,
- un anneau (21) intérieur affichant le résultat de la classification structurelle pour chaque segment de la tête du nerf optique (20) ou de l'autre structure par un code couleur, des motifs graphiques ou des niveaux de luminosité et
- un anneau (22) extérieur affichant dans le même code couleur, des motifs graphiques concordants ou des niveaux de luminosité le résultat de la classification pour la zone du champ visuel qui correspond à un segment de la tête du nerf optique (20) ou de l'autre structure.

2. Procédé selon la revendication 1, **caractérisé en ce qu'**on divise chaque segment de la tête du nerf optique (20) et chaque zone du champ visuel dans l'une des trois catégories « dans des limites normales » « à la limite » et « hors des limites normales » et/ou **en ce que** le code couleur G correspond à « dans des limites normales », le code couleur Y correspond à « à la limite » et le code couleur R correspond à « hors des limites normales ».

3. Procédé selon la revendication 1 ou 2, **caractérisé en ce qu'**on soumet le stimulus FDF représenté à une correction d'artefact, un passage progressif d'une inversion du point cible de l'état en phase dans l'état décalé en phase par rapport à des points d'arrière-plan étant réalisé.

4. Procédé selon l'une quelconque des revendications 1 à 3, **caractérisé en ce qu'**on procède à une surveillance de la fixation en temps réel, notamment pour évaluer une fatigue de la personne, pendant la réalisation de l'examen FDF, des cibles étant mises à disposition en différents endroits par rapport à l'axe optique de l'instrument ou de l'unité de génération d'images, pour la fixation de l'oeil, le caractère correct de la fixation devant être confirmé dans une période prédéfinie.

5. Procédé selon l'une quelconque des revendications 1 à 4, **caractérisé en ce que** par mise en oeuvre d'une surveillance permanente du temps de réaction, il s'effectue une évaluation de la fiabilité de la personne ou du patient, notamment un temps prédéfini entre le déclenchement d'une représentation de cible et la manoeuvre d'une touche de réponse étant prédéfini et/ou surveillé en tant que temps de réaction.

6. Procédé selon l'une quelconque des revendications 1 à 5, **caractérisé en ce qu'**on procède à un examen d'un champ de bordure, notamment au moyen de sources lumineuses placées physiquement et/ou optiquement dans un champ de bordure placé à l'horizontale et/ou **en ce qu'**un procédé de commande d'une résolution lumineuse augmentée est mis en oeuvre, notamment par utilisation d'une technologie de cartes graphiques standard, pour l'unité de génération d'image, notamment un écran noir et blanc, deux canaux couleurs étant utilisés pour la mise à disposition du nombre prédéfini de niveaux de luminosité.

7. Procédé selon l'une quelconque des revendications 1 à 6, **caractérisé en ce qu'**on utilise un algorithme de seuillage adaptatif échelonné (ASTA), notamment pour l'affectation de chaque position cible.

8. Procédé selon l'une quelconque des revendications 1 à 7, **caractérisé en ce qu'**on réalise une stratégie de limite de sauts, au niveau de l'arrière-plan ponctuel, un changement de contraste entre des représentations successives étant limité.

9. Dispositif, destiné à réaliser le procédé selon l'une quelconque des revendications 1 à 8, **caractérisé en ce qu'**un système informatique et une unité de génération d'image actionnable par celui-ci sont prévus pour la représentation des stimuli FDF.

10. Dispositif, destiné à réaliser le procédé selon la revendication 1, **caractérisé en ce que** pour la détection des structures et/ou des modifications structurelles, une source lumineuse et un système de balayage sont prévus et sont électriquement connectés de manière connue avec l'ordinateur en question, ainsi qu'avec l'unité de génération d'image en question de la revendication 9, pour la transmission de données.

# Fig. 1

HG Punkt
Leuchtphase

Weiß
MLeucht
Schwarz

1.1

Zielpunkt
+1.0 Leuchtphase

1.2

Zielpunkt
+0.5 Leuchtphase

1.3

Zielpunkt
-0.5 Leuchtphase

1.4

Zielpunkt
-1.0 Leuchtphase

1.5

# Fig. 2

13

14

1

2

11

12

4    3

# Fig. 3

# Fig. 4

# Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2002047987 A
- EP 0962188 A1
- US 5270750 A
- EP 1442695 A2
- DE 10233960 A1
- WO 0160241 A1

**In der Beschreibung aufgeführte Nicht-Patentliteratur**

- **QUAID ; FLANAGAN.** *Vision Research,* 2005, vol. 45, 1075-1084
- **RAMACHANDRAN V. S. ; ROGERS-RAMACHANDRAN D.** Phantom Contours: A New Class of Visual Patterns that Selectively Activates the Magnocellular Pathway. *Man. Bulletin of the Psychonomic Society,* 1991, vol. 29, 391